(19) Europäisches Patentamt
European Patent Office
Office européen des brevets

(11) Numéro de publication: **0 155 871**
**A1**

(12) **DEMANDE DE BREVET EUROPEEN**

(21) Numéro de dépôt: **85400322.5**

(22) Date de dépôt: **21.02.85**

(51) Int. Cl.[4]: **C 07 C 69/734**
C 07 D 213/42, C 07 D 277/04
C 07 C 67/327, C 07 C 67/00

(30) Priorité: **22.02.84 FR 8402681**

(43) Date de publication de la demande:
**25.09.85 Bulletin 85/39**

(84) Etats contractants désignés:
**AT BE CH DE FR GB IT LI LU NL SE**

(71) Demandeur: **Etablissement Public dit: CENTRE NATIONAL DE LA RECHERCHE SCIENTIFIQUE (CNRS)**
**15, Quai Anatole France**
**F-75007 Paris(FR)**

(72) Inventeur: **Boudet, Alain**
**18 rue Caubere**
**F-31400 Toulouse(FR)**

(72) Inventeur: **Cazaux, Louis**
**Vigoulet-Auzii**
**F-31320 Castanet-Tolosan(FR)**

(72) Inventeur: **Gorrichon, Liliane**
**20, rue des Cèdres**
**F-31400 Toulouse(FR)**

(72) Inventeur: **Grand, Claude**
**6, Impasse Dordac**
**F-31650 Saint-Orens de Gameville(FR)**

(74) Mandataire: **Peaucelle, Chantal et al,**
**Cabinet Plasseraud 84, rue d'Amsterdam**
**F-75009 Paris(FR)**

(54) **Composés possédant, notamment, des propriétés inhibitrices vis-à-vis de la lignification dans les végétaux et procédé pour la réduction de leur taux de lignine.**

(57) Les composés possédant des propriétés inhibitrices vis-à-vis de la biosynthèse de la lignine dans les végétaux répondent à la formule:

$$Ar\text{-}C(R_1) = C(R_2) - Y$$

dans laquelle:

– Ar représente au moins un cycle aromatique ou hétérocyclique, le cas échéant, substitué,

–$R_1$ et $R_2$, identiques ou différents l'un de l'autre, représentent un atome d'hydrogène, d'halogène ou un groupe alcoyle ou alcoxy et $Y$ un groupe fonctionnel.

EP 0 155 871 A1

"Composés possédant, notamment, des propriétés inhibitrices vis-à-vis de la lignification dans les végétaux et procédé pour la réduction de leur taux de lignine"

L'invention est relative à de nouveaux composés et compositions dotés, notamment, de propriétés inhibitrices vis-à-vis de la lignification dans les végétaux.

Elle concerne également un procédé de traitement des végétaux afin de réduire leur taux de lignine et ses applications industrielles en particulier dans le domaine de l'alimentation humaine et animale.

Les lignines sont des polymères phénoliques élaborés par les végétaux, formés de motifs monomères de trois types, à savoir d'alcools p-coumarylique, coniférylique et sinapylique.

La biosynthèse de ces motifs monomères est réalisée dans la plante, à partir de phénylalanine selon un processus comportant plusieurs étapes dans lesquelles interviennent de nombreuses enzymes.

Les deux dernières étapes de la biosynthèse des monomères qui sont spécifiques de la lignification, correspondent à des étapes de réduction et sont catalysés respectivement par la cinnamoyl :CoA réductase (désignée ci-après par CCR) et l'alcool cinnamylique déshydrogénase (désignée par CAD).

La teneur en lignines et les proportions relatives des trois monomères ainsi que leur mode de liaison sont variables d'une espèce à l'autre et selon l'âge et la nature du tissu et des cellules considérées.

D'une manière générale, les lignines sont synthétisées dans les parois des cellules telles que le xylème et le sclérenchyme, assurant par là des fonctions importantes de soutien et de conduction.

Elles constituent cependant un obstacle à l'utilisation optimale des végétaux dans divers domaines.

Ainsi, dans la nutrition animale, on constate que des taux de lignines importants, par exemple, dans les plantes fourragères, nuisent à la digestibilité des plantes par les animaux.

En outre, on observe dans certaines productions végétales une lignification intense, après la récolte, qui les rend rapidement impropres à la consommation.

Ainsi, des tubercules tropicaux tels que les ignames, utilisés pour l' alimentation humaine, ne peuvent plus être consommés au bout de quelques semaines après leur récolte en raison d'une nouvelle synthèse de parois secondaires lignifiées.

De plus, dans certaines plantes, par exemple, chez les plants de tomates, on constate un antagonisme entre la production de lignine et la croissance de la plante , une lignification rapide réduisant les possibilités de croissance cellulaire.

Diverses solutions ont été alors recherchées pour réduire les taux de lignine le terme général de lignine désigne dans la suite de la description et dans les revendications les différentes lignines évoquées ci-dessus

Par mutagénèse, ou par croisement, on a pu ainsi obtenir une hypolignification des plantes fourragères conduisant à une meilleure ingestibilité et digestibilité

par les animaux. Toutefois, leur faible productivité ne permet pas une exploitation de telles variétés à l'échelle industrielle.

On a également envisagé d'inhiber spécifiquement, par voie chimique, des enzymes impliquées dans la biosynthèse de la lignine.

Cependant, d'une manière générale, les inhibiteurs proposés présentent l'inconvénient d'agir sur d'autres constituants biochimiques importants de la plante.

L'invention a donc pour but de fournir de nouveaux inhibiteurs permettant de réduire le taux de lignine dans les plantes en agissant sur le processus de lignification sans perturber de manière significative la synthèse de constituants biochimiques importants tels que protéines, acides nucléiques, chlorophylles, polysaccharides pariétaux et autres produits.

Elle a également pour but de fournir un procédé de traitement des végétaux permettant d'abaisser leur teneur en lignine d'au moins 20 à 30%.

Elle vise, en outre, à fournir une famille d'inhibiteurs de synthèse de la lignine dont l'utilisation permet notamment d'améliorer la digestibilité des végétaux destinés à l'alimentation humaine et animale et leur conservation.

Les composés possédant des propriétés inhibitrices vis-à-vis de la biosynthèse de la lignine dans les végétaux répondent à la formule I :

$$Ar-C(R_1) = C(R_2) - Y$$

dans laquelle :

- Ar représente au moins un cycle aromatique, en particulier, un groupe phényle ou un hétérocycle, en particulier, un hétérocycle azoté tel qu'un radical

pyridyle ou pyrimidyl . le groupe aromatique ou hétérocyclique étant, le cas échéant, substitué par au moins un radical Z en position ortho,méta ou para,Z étant choisi de préférence parmi le groupe -OH, alcoxy renfermant notamment de 1 à 4 atomes de carbone, $-NH_2$, -NH (alc. ou M.), $-N-(alc.)_2$, alc. représentant un groupe alcoyle, notamment,de 1 à 4 atomes de carbone et M représentant un radical aromatique tel qu'un groupe phényle ;

- $R_1$ et $R_2$, identiques ou différents l'un de l'autre, représentent un atome d'hydrogène. d'halogène ou un groupe alcoyle ou alcoxy. de préférence de 1 à 4 atomes de carbone. et

- Y représente un groupe fonctionnel tel qu'un groupe de structure $-\underset{O}{\underset{\|}{C}}-OR$, $-\underset{O}{\underset{\|}{C}}R$, $-\underset{O}{\underset{\|}{C}}-SR$, $-\underset{O}{\underset{\|}{C}}-N(R')R$, $-CH_2OR$, $-CH(R')-OR$, $-CH{=}N-R$, R et R',identiques ou différents l'un de l'autre représentant un atome d'hydrogène,un radical alcoyle linéaire ou ramifié ,notamment de 1 à 4 atomes de carbone un radical $-(CH_2)_n-N\langle$ , n étant un entier, identique ou différent pour R et R', de préférence de 1 à 4, ou au moins un cycle aromatique ou encore un hétérocycle, notamment un radical thiazolyle ou pyridine, l'atome d'azote étant, le cas échéant, un élément de l'hétérocycle, et les dérivés de ces groupes fonctionnels.

D'une manière avantageuse, ces inhibiteurs comportent des éléments de reconnaissance du site actif des enzymes impliquées spécifiquement dans le processus de lignification et entrent ainsi en compétition avec le substrat des enzymes au niveau de sa fixation.

L'activité de ces composés est d'autant plus forte que leurs éléments structuraux les rapprochent des substrats de l'enzyme à inhiber.

Un groupe préféré de dérivés mis en oeuvre selon l'invention comprend au moins un dérivé de formule I dans laquelle Y représente un groupe ester.

Le substituant Y, dans un autre groupe, présente la structure -CH=N-R.

Dans un autre groupe de produits, Y représente un radical -C-SR. Des dérivés de ce radical, par exemple, des groupes de structure $-S-CH_2-CH_2-NH-OCO-R$ constituent des produits intéressants.

Un autre groupe avantageux renferme au moins un dérivé de formule I dans laquelle Y représente un éther d'alcool primaire ou seconaire.

Dans encore un autre groupe préféré Y représente un dérivé de cétone.

Un autre groupe préféré encore comprend, comme substituant Y, un radical amide de structure $-\underset{\underset{O}{\|}}{C}-N(R'')R$. Des produits préférés de ce type comprennent un substituant Y représentant un groupe -CO-NH-hétérocycle, en particulier -CO-NH- 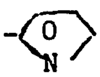 ou -CO- 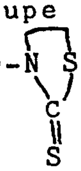

Des dérivés de ces différents groupes. particulièrement préférés en raison de leur action inhibitrice élevée vis-à-vis de la CAD comprennent des substituants $R_1$ et $R_2$ différents l'un de l'autre.

Dans des composés de ce type. $R_1$ représente avantageusement un atome d'halogène (F.Cl.Br ou I). ou encore un groupe alcoyle et $R_2$ représente un atome d'hydrogène.

D'autres dérivés de ces différents groupes dotés d'une grande efficacité pour moduler en baisse le taux de lignine de végétaux comprennent des substituants $R_1$ et $R_2$ identiques.

Des composés avantageux de ce type comportent des substituants $R_1$ et $R_2$ représentant tous deux de l'hydrogène.

Indépendamment des significations préférées de Y,$R_1$ et $R_2$ mentionnées ci-dessus, ou le cas échéant, en plus de ces significations, des composés préférés renferment selon l'invention un groupe Ar représentant au moins un groupe phényle.

D'autres composés présentant également un intérêt au regard des applications envisagées renferment un groupe Ar comportant au moins un hétérocycle. Des hétérocycles conduisant à des produits de grande efficacité comprennent des groupes pyridyle,pyrimidyle ou leurs dérivés tels que le groupe nicotinamide.

En ce qui concerne le groupe -Ar, il s'agit d'un groupe non substitué ou de préférence substitué par un ou encore plusieurs groupes Z, en position ortho, ortho', méta, méta' et/ou para, identiques ou différents les uns des autres.

Un groupe Ar préféré comporte un substituant Z en para et répond à la formule

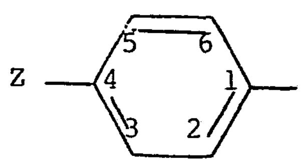

D'autres produits dotés d'une efficacité inhibitrice élevée comprennent un groupe Ar substitué en outre en méta et méta' (à savoir sur les positions 3 et 5),de formule :

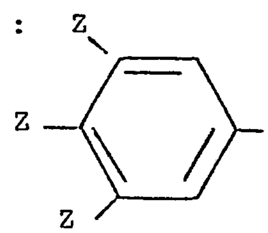

Dans d'autres produits encore, Ar représente un radical de formule :

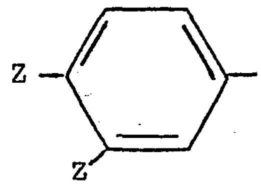

Des significations préférées de Z correspondent à un groupe-hydroxy en position para et alcoxy en position méta et/ou méta',notamment,méthoxy.

Les composés correspondants dans lesquels Ar comprend, au lieu d'un groupe phényle, un hétérocycle, en particulier, un hétérocycle azoté tel q'un cycle pyridyle ou pyrimidyle, constituent également des composés avantageux.

Les dérivés de formule I dans laquelle Y représente un groupe 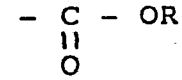 sont préparés par condensation d'un énolate de formule II avec un aldéhyde aromatique de formule III :

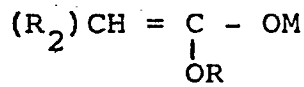

(II)

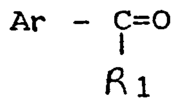

(III)

ce qui conduit à un dérivé de formule

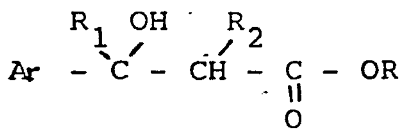

qui est soumis, au cours de l'étape suivante, à une réaction de déshydratation permettant d'obtenir le produit de formule I recherché.

Dans ces formules Ar, R, $R_1$ et $R_2$ présentent les significations données ci-dessus et M représente un cation métallique.

L'étape de condensation est avantageusement réalisé à une température voisine de 0°C en milieu solvant anhydre. Un solvant du type du tétrahydrofuranne convient à cet effet. L'énolate mis en oeuvre est obtenu par réaction de l'ester avec un dérivé organométallique. Cette réaction est avantageusement réalisée à température inférieure de préférence à -50°C, et en milieu solvant anhydre.

Pour l'étape de déshydratation, on utilise un agent de déshydratation du type de chlorure de thionyle

Le rendement élevé de ces étapes, voire pratiquement quantitatif, confère un grand intérêt à cette voie de synthèse.

Ce mode opératoire est également avantageusement applicable à la préparation des dérivés dans lesquels

Y représente un groupe cétone ou amide. On met alors en oeuvre dans le procédé ci-dessus la cétone ou l'amide approprié à la place de l'ester.

Des produits dans lesquels Y représente un groupe $- CH = N - R$ sont obtenus par exemple par addition d'une amine $NH_2R$ sur l'aldéhyde aromatique, suivie d'une déshydratation.

En variante, on utilise comme produit de départ des acides de formule :

$$Ar - C(R_1) = C(R_2) - \underset{\underset{O}{\|}}{C} - OH$$

et on forme le sel d'iminium de ces acides de formule :

$$Ar - C(R_1) = C(R_2) - \underset{\underset{OH}{|}}{C} = \overset{+}{N}\begin{matrix} R' \\ R'' \end{matrix} \quad X^-$$

$X^-$ représentant un anion.

Dans ces formules, Ar, $R_1$ et $R_2$ présentent les significations données ci-dessus et R' et R" identiques ou différents l'un de l'autre, représentent avangageusement un groupe alcoyle ou acyle.

Pour former ces sels d'iminium, on opère avantageusement selon le procédé décrit par Böhme et Viehe dans Iminium Salt in organic chemistry in Advanced organic chemistry - Ed - E.C. Taylor - Vol 9 - Part 1 et 2 - John Wiley and sons - N.Y. 1976 et 1979.

Les sels d'iminium sont préparés en opérant par exemple selon la méthode décrite dans Tetrahedron Letters, 1983, vol. 24, n° 14, p. 1543.

En traitant le sel d'iminium de l'acide de départ par un excès d'alcool, on obtient l'ester correspondant.

Pour préparer des dérivés comportant un groupe terminal amide, à la place du groupe ester, on opère avantageusement comme décrit ci-dessus, mais on utilise un dérivé métallique d'amide, tel qu'un dérivé lithié.

De même, les éthers sont élaborés en utilisant un excès d'alcool linéaire ROH, ou une quantité stoéchiométrique, et les thioléthers, à partir de RSH.

Les composés de synthèse définis ci-dessus possèdent un effet inhibiteur élevé vis-à-vis de la production de lignine.

L'étude effectuée in vitro concernant l'action de ces produits a montré leur forte action inhibitrice vis-à-vis de la CAD purifiée. Compte-tenu des résultats obtenus en étudiant des composés diversement substitués, il apparaît que ces composés agissent comme des inhibiteurs du type analogues de substrats. Leur action inhibitrice est donc réversible.

Les résultats obtenus in vitro ont été avantageusement confirmés in vivo. On mesurera l'intérêt de cette action in vivo, qui ne pouvait être prévisible, ainsi qu'il est bien connu dans ce domaine, sur la base des résultats observés in vitro. Les travaux effectués à cet égard ont montré une action sur la synthèse des flavonoïdes conduisantà une réduction de leur production. Cependant, la synthèse des constituants biochimiques principaux n'apparaît pas affectée dans son ensemble de manière significative. On n'observe pas en outre d'effet sur les métalloenzymes des végétaux autres que la CAD.

L'invention vise donc l'utilisation de ces composés pour réduire le taux de lignine des végétaux

selon laquelle on met en oeuvre une quantité efficace d'au moins un composé de formule I.

Ces compositions d'inhibiteurs, selon l'invention, se présentent avantageusement sous forme de poudre à diluer au moment de l'emploi, ou de solutions, de préférence de solutions aqueuses renfermant ces inhibiteurs.

Les préparations sous forme de solutions auqeuses renferment avantageusement les inhibiteurs en question à raison d'au moins 200 µM. Des préparations préférées renferment de 200 µM à 500µM, avantageusement de 200 à 300µM.

Ces préparations peuvent, en outre, renfermer d'autres ingrédients utiles à la plante, notamment, des éléments nutritifs et/ou des agents de traitement.

L'application est réalisée de préférence dans la phase ou les phases de lignification intense du végétal. On opère par pulvérisations, arrosage ou autre mode approprié au type de végétal traité.

Compte-tenu de la sensibilité de l'action des composés de l'invention, on renouvelle avantageusement le traitement à plusierus reprises.

Une pulvérisation, trois à quatre fois, par exemple, à environ 15 jours d'intervalle, sur la production végétale, au moment de la phase de croissance du végétal, permet de réduire le taux de lignine de manière satisfaisante. Ce traitement est utilisable sur des espèces végétales variées telles que, par exemple, le peuplier, le maïs, les plantes à tubercules et autres productions végétales.

Dans le cas de plantes fourragères, il en résulte une ingestibilité et une digestibilité accrues de ces dernières par les ruminants,permettant ainsi une augmentation de la productivité.

Le procédé de l'invention revêt également un intérêt essentiel dans l'alimentation humaine en permettant de réduire le taux de lignines dans les productions végétales comme évoqué ci-dessus. Une pulvérisation au moment du stockage des végétaux renouvelée à une semaine d'intervalle environ deux à toutefois, s'avère généralement satisfaisante.

D'autres caractéristiques et avantages de l'invention apparaîtront dans les exemples qui suivent donnés à titre illustratif.

EXEMPLE 1 : Préparation de dérivés de formule I dans laquelle Y représente un groupe $-\underset{\underset{O}{\|}}{C}-OR-$

On prépare le férulate de tertiobutyle (FTB) de formule

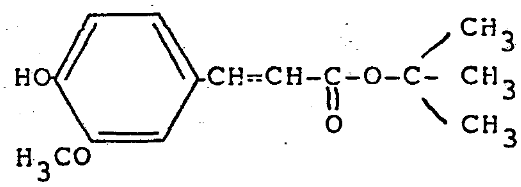

par condensation, au cours d'une première étape a), de l'énolate de l'ester tertio-butylique avec la vanilline, suivie d'une déshydratation, durant une deuxième étape b), du produit de condensation formé.

Ces étapes sont réalisées comme suit :

a) A une solution de 10mM d'isopropyl-amidure de lithium en solution dans 10 ml de tétrahydrofuranne (THF), on ajoute à 78°C, goutte à goutte, 10 mM d'acétate de tertiobutyle.

On maintient 15 mn à -78°C à l'abri de l'air et de l'humidité. On ajoute ensuite à l'aide d'une seringue, goutte à goutte, 4mM de vanilline dans 8ml de THF et on amène la température à 0°C. Après 1 heure sous agitation, on hydrolyse à l'aide de $NH_4Cl$ en solution saturée dans l'eau. On ajoute 10 ml et on extrait par l'éther (2 fois 20 ml). Après décantation et séchage sur carbonate de potassium, on élimine le solvant (rendement en β -hydroxy ester : 85%).

b) A 4 mM de 50 $Cl_2$, en solution dans 4 mM de pyridine, on ajoute 2mM du β -hydroxyester obtenu à l'étape précédente. Il se forme un mélange visqueux brun qu'on maintient sous agitation 5 à 10 mn. On procède ensuite à une hydrolyse et une extraction par l'éther en opérant comme dans l'étape a). Le rendement

est quantitatif. ($R_F$ = 0,66 (chloroforme avec 5 % de méthanol) ; $R_F$ du produit de départ : 0,46).

En opérant comme décrit ci-dessus, mais en utilisant à la place de l'ester, le dérivé de cétone et de thiol , on obtient les dérivés correspondants dans lesquels le groupe $-\underset{\underset{O}{\|}}{C}-O-$tertio-butyle est remplacé respectivement par le groupe $-\underset{\underset{O}{\|}}{C}-$tertio-butyle et $-\underset{\underset{O}{\|}}{C}-S-$tertio-butyle.

Dans d'autres essais, on utile à la place de la vanilline, des aldéhydes aromatiques de formule :

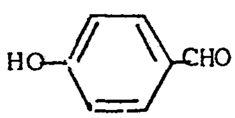 ou 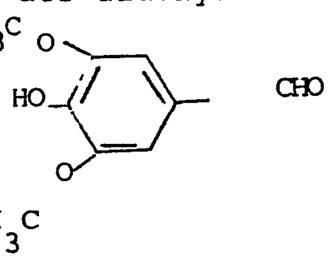

EXEMPLE 2 : Variante de préparation du FTB :

On ajoute à 0°C, le chlorure de l'acide oxalique (0,5 ml) à une solution de diméthylformamide (DMF) distillée et séchée sur tamis (2mM; 0,154 ml) dans 3 ml de $CH_2Cl_2$ anhydre.

On maintient l'agitation pendant 1 heure puis on évapore le solvant à l'abri de l'humidité. On obtient une poudre blanche.

A -30°C, on ajoute à ce résidu, 3 ml d'acétonitrile, 5ml de THF anhydre et une solution de 2mM d'acide férulique, de 2mM de pyridine dans 3ml de THF. L'addition se fait goutte à goutte. Le mélange réactionnel devient jaune. On maintient l'agitation pendant 1 h et on ajoute, à - 30°C, un excès de méthanol (4ml) goutte à goutte.

On maintient l'agitation durant 30 mn puis on hydrolyse à l'aide de HCl 0,5N, on extrait à l'éther et on

neutralise par du bicarbonate de sodium à 5%. Le $R_f$ dans $CH_2Cl_2$ renfermant 5% de méthanol est de 0,63 et le rendement de 96%.

EXEMPLE 3 : Préparation d'amides des acides cinnamiques :

On opère selon le schéma réactionnel ci-dessous :

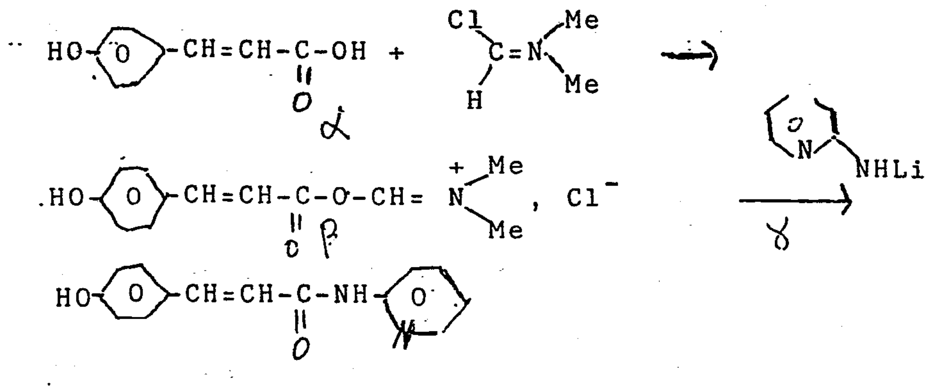

α) Les composés α à δ indiqués sur ce schéma sont préparés respectivement selon les étapes suivantes :

- A 0°C, sous azote et agitation magnétique, 4 mmoles (1ml) de chlorure d'oxalyle sont ajoutées goutte à goutte à la seringue à travers un septum à une solution de 4mmoles de DMF anhydre (0,29 g;031 ml) dans 6 ml de $CH_2Cl_2$. A la fin du dégagement gazeux, l'agitation est maintenue 1 h à 0°C Après élimination du solvant, on obtient une poudre blanche très sensible à l'humidité.

β) Préparation du chlorure de carboxy méthylèniminium

La poudre est mise en solution dans 16 ml de THF anhydre. On ajoute à -30°C goutte à goutte une solution de 3,6 mmoles d'acide (0,7 g de 1a, 0,85 g de 1b), de 4 mmoles de pyridine (0,32g) et de 6 ml deTHF. Le milieu réactionnel est soumis à agitation 1 heure à - 30°C et devient d'une consistance visqueuse et d'une couleur jaune pâle. (1a et 1b correspondent respectivement à l'acide férulique et à l'acide O-acétyl férulique).

γ) Préparation de l'amino-pyridine lithiée :

On opère à -30°C à partir de 12 mmoles (7,5ml) de butyllithium (1,6M dans l'hexane) et de 12 mmoles (1,13 g)

de 2-aminopyridine dans 12 ml de THF (agitation pendant 30°C.

δ) Sur le lithium on verse le sel d'iminium préparé ci-dessus sous azote, par l'intermédiaire d'un coude de verre rodé reliant les deux réacteurs.

Le milieu réactionnel est réchauffé à la température ambiante et l'agitation est maintenue pendant 2 heures.

Après hydrolyse, et extraction à l'acétate d'éthyle, le DMF formé est distillé.

Le produit brut réactionnel (1 g) est purifié en chromatographie sur colonne (éluant $CH_2Cl_2$/acétate d'éthyle 9/1) et, si nécessaire, en HPLC. On obtient après purification 196 mg ; 20% de produit attendu.

Les produits Ar 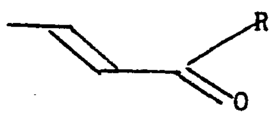 R rapportés dans le tableau suivant ont été préparés en opérant selon les exemples ci-dessus.

| | Ar | R | Rdt % |
|---|---|---|---|
| (hydroxy-4 méthoxy-3 phényl)-3N(pyridyl-2)propène 2 amide | 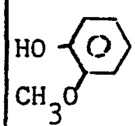 | 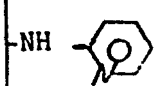 | 20 FAP |
| (méthoxy-4 phényl)-3 N(pyridyl-2)propène 2 amide | 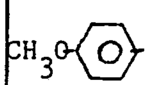 | 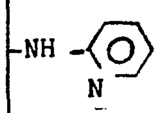 | 80 |
| (hydroxy-4 méthoxy-3 phé-nyl)-3 | 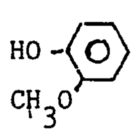 | 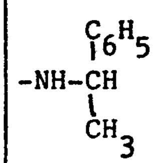 | 72 FMT |

EXEMPLE 4 :

- Synthèse du N[hydroxy-4 méthoxy-3 phényl) 3 propène-2oyl] thioxo 2 thiazolidine ou FMT, de formule :

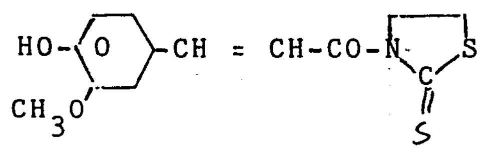

Ce composé a été préparé selon la méthode de Fujita et al dans J.C.S. Perkin I,11, 2470-2573,1980, soit en utilisant le dicyclocarbodimide, soit à partir du chlorure de l'acide ferulique (le groupe hydroxyle est protégé) en présence de sel de thallium.

EXEMPLE 5 : Action in vitro d'inhibiteurs selon l'invention sur la lignification de végétaux.

Les études dont les résultats sont rapportés ci-après ont été effectuées sur de jeunes plants de peuplier et de maïs, variété INRA 400, obtenus en salle conditionnée (température maintenue à 22°C, humidité de 60 à 80% de saturation, éclairement de 10 000 lux , héméropériode de 12 heures).

Les plants de peuplier sont obtenus à partir de boutures de l'année (segments de tiges de 15 cm), placées dans des bacs contenant de la vermiculite et arrosées quotidiennement par une solution nutritive riche en azote présentant la composition suivante :

| Composés | Concentrations g/l |
|---|---|
| $KNO_3$ | 0,303 |
| $KH_2PO_4$ | 0,102 |
| $K_2HPO_4$ | 0,043 |
| NaCl | 0,011 |
| $Ca(NO_3)_2$ | 0,410 |
| $Ca(NO_3)_2,H_2O$ | 0,455 |
| $Ca(NO_3)_2,4H_2O$ | 0,590 |
| $MgSO_4$, $7H_2O$ | 0,184 |
| $NH_4NO_3$ | 0,160 |
| Séquestrène de fer | $0,59x10^{-3}$ |
| $MnSO_4$, $H_2O$ | $1,69 \times 10^{-3}$ |
| $CuSO_4$, $5H_2O$ | $0,25 \times 10^{-3}$ |
| $ZnSO_4$, $7H_2O$ | $0,29 \times 10^{-3}$ |
| $H_3BO_4$ | $1,86 \times 10^{-3}$ |
| $(NH_4)_6O_{24}Mo_7.4H_2O$ | $0,035 \times 10^{-3}$ |

Les échantillonnages ont été effectués à partir de jeunes plants de peuplier âgés de 3 mois et comportant entre 20 et 25 entrenoeuds.
Les fractions tissulaires ont été obtenues par grattages successifs au scalpel selon la méthodologie décrite par GRAND, thèse de 3ème cycle, 1979, Université de Toulouse.
Dans tous les cas, le végétal est stabilisé dans l'azote liquide, puis lyophilisé pendant 48 h et conservé à -20°C dans des flacons colorés et bouchés.

Les semences de maïs sont mises à imbiber pendant 14 heures dans l'eau distillée, à la température ambiante, puis enfouies à 1 cm de profondeur dans la vermiculite répartie dans des bacs en polyéthylène.
Les bacs sont arrosés quotidiennement par une solution nutritive présentant la composition suivante :

| Composés | Concentrations g/l |
|---|---|
| $KNO_3$ | 1.01 |
| $KH_2PO_4$ | 0.27 |
| $MgSO_4 . 7 H_2O$ | 0.96 |
| $Ca(NO_3)_2 . 4 H_2O$ | 2.36 |
| $FeCl_3$ | $0.97 \times 10^{-3}$ |
| EDTA ($Na_2$) | $1.12 \times 10^{-3}$ |
| $H_3BO_3$ | 0.35 |
| $MnSO_4 . H_2O$ | 0.35 |
| $CuSO_4 . 5 H_2O$ | 0.07 |

En vue du dosage des lignines, le matériel végétal est préparé selon la méthode décrite par ALIBERT et BOUDET dans Physiol.vég.17, 75-82, 1979.
50 à 500 mg de végétal lyophilisé, réduit en poudre au broyeur àbilles Dangoumeau (Prolabo) est extrait par 10 ml d'eau distillée (3 fois), soumis à agitation 5 mn, puis à une opération de centrifugation durant 5 mn à 3000 g.
On récupère le culot de centrifugation puis on l'extrait à l'aide de 10 ml de NaCl M, 2% en Triton X 100 (3 fois) et on soumet le mélange à agitation 5 mn. Après une centrifugation de 5 mn, à 3000 g, on récupère le culot qu'on extrait en continu par un mélange alcool-benzène (½ ; v/v) avec un débit de $1ml.mn^{-1}$ (1 l/g de matériel végétal).. Le résidu obtenu est rincé avec de l'éthanol absolu et séché sous vide.
La préparation obtenue, appelée "poudre alcool-benzène" correspond à une fraction pariétale enrichie en lignines et polysaccharides (cellulose, hémicelluloses).
Les acides cinnamiques, liés aux lignines sous formes d'esters. sont libérés par hydrolyse des "poudres alcool-benzène" par de la soude 2N pendant 1 heure sous atmosphère d'azote.
Après réacidification à pH2 par de l'acide chlorhydrique 3N et centrifugation, le surnageant contenant les acides cinnamiques est séparé du résidu. Les "poudres alcool-benzène" ainsi hydrolysées sont lavées par de l'eau distillée jusqu'à pH neutre et séchées, sous vide dans un dessiccateur en présence de silica -gel.
Les teneurs en lignines sont estimées par la méthode

gravimétriques de KLASON (Tappi Standard method T12m) modifiée par EFFLAND décrite dans Tappi, 50, 143-144) On rapporte dans le tableau suivant les résultats obtenus concernant l'effet mesuré in vitro d'inhibiteurs selon l'invention à différentes concentrations vis-à-vis de la CAD de peuplier isolée selon la méthode de SARNI et al, dans, EUROP JOURNAL OF BIOCHEMISTRY, 139, P.259-265, 1984.

| Composé | | Inhibition % à 2mM | | |
|---|---|---|---|---|
| p-Coumarate de tertiobutyle (PCB) | 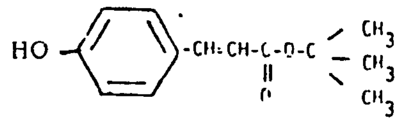 | 40 | | |
| Ferulate de tertiobutyle (FTB) | 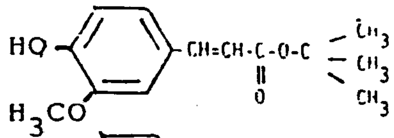 | 75 | | |
| Sinapate de tertiobutyle (STB) | 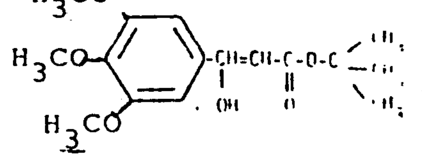 | 55 | | |
| | | inhibition à 0,062 | 0,125 | 0,250mM |
| FAP = | 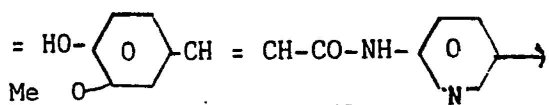 | 28 | 100 | |
| FMT = | 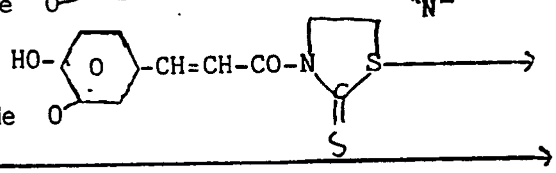 | 0 | 66 | 100 |
| FTB | | 29 | 63 | 100 |

Les mesures des trois derniers résultats ont été réalisées dans le sens physiologique c'est-à-dire en suivant la réduction de l'aldéhyde coniférylique(oxydation du NADPH mesurée à 340 nm).

Cette inhibition mesurée in vitro dans le sens physiologique (pH 6,5) est 10 à 15 fois plus importante que dans le sens inverse, c'est-à-dire alcool ⟶ aldéhyde (pH 8,8).

Ces résultats montrent l'efficacité d'inhibiteurs de l'invention.

Le dérivé désigné FTB dans lequel le noyau aromatique est substitué en para et en méta permet d'obtenir des taux d'inhibition d'environ 75 %.

Les dérivés correspondants dans lesquels la chaîne est saturée donnent respectivement des taux d'inhibition de 15, 20 et 25 %. Les meilleurs résultats obtenus avec l'insaturation éthylènique confirme l'analogie d'action avec les substrats naturels de l'enzyme.

L'étude in vitro, dans les conditions rapportées ci-dessus, de dérivés comportant un hétérocycle azoté et/ou des groupes terminaux éther, alcool, thiols, cétone, amide ou acide, a également confirmé l'efficacité de ces produits vis à vis de l'inhibition de la CAD.

L'étude des modalités d'action des inhibiteurs de l'invention montre la réversibilité de leur action. Ainsi, la dialyse d'un mélange enzyme - FTB, à la concentration de 2 mM contre un tampon tris/HCl 0,1 M, pH 7,5, pendant 14 heures à 4°C et la mesure avant et après dialyse de l'activité de CAD, montre une restitution totale de l'activité CAD.

L'étude de leur action sur différentes enzymes a également été réalisée.

On a étudié ainsi l'action d'inhibiteurs selon l'invention sur des enzymes du métabolisme phénolique telles que la phénylalanine ammonialyase (PAL), la catéchol O-méthyl transférase (OMT), l'hydroxycinnamate : CoA ligase (HCl) et la cinnamoyl : CoA réductase (CCR), et des enzymes végétales à zinc, telles que l'éthanol déshydrogénase (EDH), la superoxyde dismutase (SOD) et l'anhydrase carbonique (CA).

Les résultats obtenus, par exemple avec le FTB montre qu'il n'agit pas sur l'EDH, la SOD et l'AC. En revanche, il s'avère actif, mis à part la CAD, vis à vis de la PAL et de la CCR.

EXEMPLE 6 : Action in vivo d'inhibiteurs selon l'invention sur la lignification.

On rapporte ci-après les résultats obtenus avec les composés du tableau ci-dessus.

Pour évaluer l'effet de ces inhibiteurs sur le processus de lignification, on a étudié notamment leur action :

1) sur le taux d'incorporation d'un traceur radioactif correspondant à un précurseur des lignines, à savoir l'acide cinnamique ($^{14}C_3$), dans des fractions de xylène de peuplier isolées à partir de tiges de peuplier (ce qui représente un moyen rapide d'évaluer le flux de synthèse des lignines sur un système simplifié) et dans les parties aériennes du peuplier (ces expériences de plus longue durée conservent un caractère de mesure dynamique, se rapprochant des conditions physiologiques).

2) sur les quantités de lignines formées par des cultures de plusieurs semaines de plants de maïs, réalisées en présence d'inhibiteurs. Dans ce cas, l'effet est observé sur l'accumulation du polymère dans des conditions naturelles.

1)- Etude de l'effet des inhibiteurs sur la synthèse des lignines radioactives à partir d'acide cinnamique ($^{14}C_3$).

a) Dans des fractions de xylème isolées à partir de tiges de peuplier.

L'acide cinnamique (149mCi/mmole,CEA) est apporté aux fractions tissulaires isolées à partir de jeunes tiges de peuplier selon la technique de GRAND décrite dans la thèse de 3ème cycle mentionnée ci-dessus.

Les tissus de xylème isolés (300 mg par essai) sont mis en suspension dans des fioles d'erlenmeyer (25ml), bouchées par du coton cardé contenant :

- du tampon Tris/HCl, 0,1M, pH 7,5 (qsp 5ml) maintenant le pH entre 7 et 8,
- du Dextran T40 (25 g/l) utilisé comme agent osmotique, ne constituant pas une source de carbone pour la cellule végétale,
- du $\beta$ mercaptoéthanol (0,1mM), antioxydant qui empêche un brunissement très rapide des tissus,
- de l'éther monométhylique de l'éthylène glycol (EMMEG) (50 µl) ou des inhibiteurs solubilisés dans ce polyol (50 µl),
- de l'acide cinnamique ($^{14}C_3$) (2,5 µCi).

Les fioles sont placées à 26°C, sous agitation orbitale (agitateur New Brunswick modèle G 10,100 rpm) et sous un éclairement de 4000 lux pendant 30 h. La radioactivité incorporée par les lignines est alors estimée à partir de "poudre alcool-benzène" obtenu par extraction à l'alcool-benzène des fractions tissulaires lyophilisées et réduites en poudre.

Afin de mesurer l'absorption de l'acide cinnamique par les fractions tissulaires et de corriger d'éventuelles différences d'absorption selon les tissus, une fraction aliquote du milieu d'incubation (50 µl) est prélevée à différents temps et la radioactivité estimée au compteur à scintillation liquide.

b) dans les tiges de peuplier -

Afin de vérifier chez des tiges de peuplier l'action de ces inhibiteurs, on réalise des incorporations d'acide cinnamique (5 µCi) à des rameaux comportant 12 entrenoeuds selon la technique de la tige sectionnée décrite par BOUDET dans C.R. Acad. Sci. Paris, P 1966-1968, 269, 1969.

La radioactivité des lignines est mesurée après 96 heures de métabolisation.

Lorsque la métabolisation est effectuée en présence des inhibiteurs, on observe une diminution de la radioactivité incorporée dans les lignines.

2- Effet des inhibiteurs sur des plants de maïs -

Des plants de maïs ont été traités pendant 10, 20 ou 30 jours par les inhibiteurs de l'invention à différentes concentrations.

Les inhibiteurs sont ajoutés au milieu nutritif.

Les cultures sur vermiculite (20 plants) sont arrosées quotidiennement par 250 ml de milieu (avec ou sans inhibiteurs). Les semis constituent le temps 0 de la culture, l'addition des inhibiteurs au milieu nutritif est réalisée dès l'émergence de la partie aérienne (2 à 3 jours). Les différentes caractéristiques des plants sont estimées après 10, 20 ou 30 jours.

A1) Influence du traitement sur la croissance des plants de maïs -

On ne note aucune différence significative entre les plants traités et les plants témoins.

A2) Influence du traitement sur les teneurs en lignines -

Après hydrolyse par la soude des "poudres alcool-benzène" dans des conditions qui éliminent les acides cinnamiques estérifiés à la paroi, on estime les teneurs en lignines par la méthode de KLASON.

Les résultats obtenus montrent que le traitement des plants par ces inhibiteurs conduit à une baisse significative des teneurs en lignines. Ainsi pour une concentration de 300 µM et 30 jours de culture, le FTB entraîne une inhibition d'environ 40 à 50 %.

A3) Etude de la spécificité d'action des inhibiteurs in vitro -

Ces études ont été réalisées sur des plants de maïs âgés de 30 jours et traités avec FTB, PCB et STB.

Les teneurs en protéines solubles, chlorophilles, polysaccharides pariétaux et acides nucléiques n'apparaissent pas significativement affectées. Ces composés étant reconnus d'une manière générale comme des "marqueurs" du métabolisme général, ces résultats montrent que les inhibiteurs de l'invention possèdent une action relativement spécifique sur le processus de lignification.

Des résultats analogues ont été obtenus avec les inhibiteurs de formule (I) dans laquelle Ar représente un hétérocycle, en particulier, un hétérocycle azoté pyridyle ou pyrimidyle.

Les composés dans lesquels Z représente un atome d'halogène ou un groupe alcoxy ont également donné des résultats satisfaisants.De même, l'utilisation de divers groupes esters, acides,amides, thiols, cétones amines, éthers et alcools primaire et secondaire correspondants, et de leurs dérivés, sur la chaîne latérale permet d'obtenir des inhibiteurs efficaces.

L'ensemble des résultats ci-dessus met en évidence les propriétés avantageuses des inhibiteurs de l'invention.

L'étude de cette famille d'inhibiteurs a également montré qu'ils n'affectaient pas sensiblement les autres constituants biochimiques de la plante, ce qui ne pouvait être prévisible sur la base des résultats in vitro.

L'efficacité de ces inhibiteurs qui se sont révélés de plus dépourvus de toxicité pour les plantes, est donc avantageusement mise à profit, selon l'invention, pour moduler en baisse la lignification des végétaux, en vue de leur utilisation dans divers domaines.

L'étude de la digestibilité par les animaux de plantes fourragères telles que fétuques, luzerne hypolignifiées montre une meilleure assimilation du végétal, ce qui permet d'augmenter la productivité.

On a également augmenté la conservation de divers végétaux traités pendant leur culture à l'aide des inhibiteurs de l'invention.

Des productions vététales utilisables pour l'alimentation humaine, qui habituellement deviennent impropres à la consommation deux ou trois semaines après leur récolte, peuvent être ainsi conservées plusieurs mois.

D'une manière générale, l'invention fournit donc les moyens de valoriser la biomasse végétale, en réduisant le taux de lignine qui constitue quantitativement dans les végétaux le second polymère après la cellulose.

REVENDICATIONS

1.- Composés possédant des propriétés inhibitrices vis-à-vis de la biosynthèse de la lignine dans les végétaux répondant à la formule I :

$$Ar-C(R_1) = C(R_2) - Y$$

dans laquelle :

- Ar représente au moins un cycle aromatique en particulier un groupe phényle ou un hétérocycle, en particulier, un hétérocycle azoté tel qu'un radical pyridyle ou pyrimidyle, le groupe aromatique ou hétérocyclique étant, le cas échéant, substitué par au moins un radical Z en position ortho, méta ou para, Z étant choisi de préférence parmi le groupe -OH, alcoxy renfermant notamment de 1 à 4 atomes de carbone, $-NH_2$, -NH (alc. ou M)-N-(alc.)$_2$, alc. représentant un groupe alcoyle, notamment de 1 à 4 atomes de carbone et M représentant un radical aromatique tel qu'un groupe phényle ;

- $R_1$ et $R_2$, identiques ou différents l'un de l'autre, représentent un atome d'hydrogène, d'halogène ou un groupe alcoyle ou alcoxy, de préférence de 1 à 4 atomes de carbone, et

- Y représente un groupe fonctionnel tel qu'un groupe de structure $-\underset{\underset{O}{\|}}{C}-OR$, $-\underset{\underset{O}{\|}}{C}R$, $\underset{\underset{O}{\|}}{C}-SR$, $-\underset{\underset{O}{\|}}{C}-N(R')R$, $-CH_2OR$, -CH(R')-OR, -CH=N-R, R et R' identiques ou différents l'un de l'autre représentant un atome d'hydrogène, un radical alcoyle linéaire ou ramifié, notamment de 1 à 4 atomes de carbone, un radical $-(CH_2)_n-N<$, n étant un entier, identique ou différent pour R et R', de préférence de 1 à 4, ou au moins un cycle aromatique ou encore un hétérocycle, notamment un radical thiazolyle ou pyridine, l'atome d'azote étant, le cas échéant, un élément de l'hétorocycle, et les dérivés de ces groupes fonctionnels.

2.- Composés selon la revendication 1, caractérisés en ce que $R_1$ représente un atome d'halogène et $R_2$, un atome d'hydrogène.

3.- Composés selon la revendication 1, caractérisés en ce que $R_1$ et $R_2$ représentent de l'hydrogène.

4.- Composé selon la revendication 1, caractérisé en ce qu'il s'agit :

- du férulate de tertiobutyle de formule :

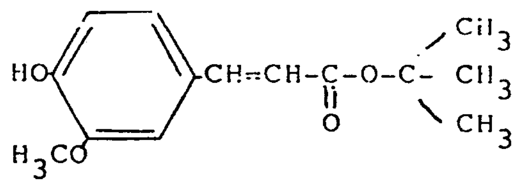

- du férulate d'amidopyridine de formule

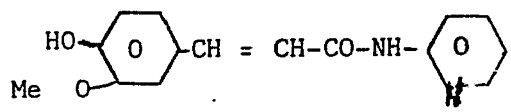

- du FMT de formule :

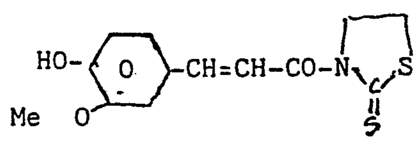

5.- Procédé de préparation de composés selon la revendication 1, caractérisé en ce que lorsque Y représente un groupe $-\underset{\underset{O}{\parallel}}{C}-OR$, on effectue la condensation d'un énolate de formule II avec un aldéhyde aromatique de formule III :

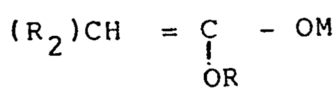

(II)

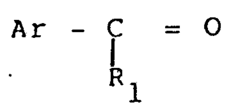

(III)

ce qui conduit à un dérivé de formule

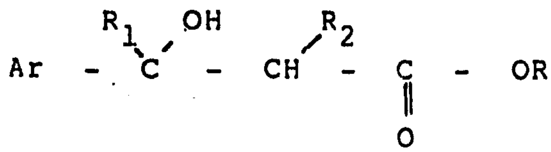

qui est soumis, au cours de l'étape suivante, à une réaction de déshydratation permettant d'obtenir le produit de formule I recherché, Ar, R, $R_1$ et $R_2$ présentant dans ces formules les significations données ci-dessus et M représentant un cation métallique, ou lorsque Y représente un groupe cétone ou amide, on met en oeuvre dans la réaction ci-dessus la cétone ou l'amide approprié à la place de l'ester, ou lorsque Y représente un groupe -CH = N-R, on fait réagir une amine $NH_2$ R avec un aldéhyde aromatique puis on soumet le produit résultant à une réaction de déshydratation, ou selon une variante, pour préparer les produits dans lesquels Y représente un ester, on utilise comme produit de départ des acides de formule :

$$Ar - C(R_1) = C(R_2) - \underset{\underset{O}{\|}}{C} - OH$$

on forme, en opérant de manière en soi connue, le sel d'iminium de ces acides de formule :

$$Ar - C(R_1) = C(R_2) - \underset{\underset{OH}{|}}{C} = \overset{+}{N}\begin{matrix} R' \\ R'' \end{matrix} \quad X^-$$

$X^-$ représentant un anion.

Ar, $R_1$ et $R_2$ présentant dans ces formules les significations données ci-dessus, R' et R" identiques ou différents l'un de l'autre, représentant avantageusement un groupe alcoyle ou acyle, puis on fait réagir le sel d'iminium de l'acide de départ avec un excès d'alcool, ce qui conduit à l'ester correspondant, ou pour préparer des dérivés comportant un groupe terminal amide, à la place du groupe ester, on opère comme décrit ci-dessus, mais on utilise un dérivé métallique d'amide, tel qu'un dérivé lithié, ou pour préparer les éthers correspondants on utilise un excès d'alcool linéaire ROH, ou une quantité stoéchiométrique, les thioéthers étant obtenus à partir de RSH.

6.- Compositions pour réduire le taux de lignine dans les végétaux caractérisées en ce qu'elles renferment une quantité efficace d'au moins un composé selon l'une quelconque des revendications 1 à 4, le cas échéant, en combinaison avec des agents nutritifs ou de traitement des végétaux.

7.- Compositions selon la revendication 6, caractérisées en ce qu'elles se présentent sous forme de poudres à diluer ou de solutions aqueuses.

8.- Compositions selon la revendication 7, caractérisées en ce qu'elles se présentent sous forme de solutions aqueuses renfermant au moins 200 µM d'au moins un composé selon l'une quelconque des revendications 1 à 7, de préférence de 200 à 500 µM.

9.- Application des compositions selon l'une quelconque des revendications 6 à 8 au traitement des plantes fourragères et des productions végétales.

Office européen des brevets

**RAPPORT DE RECHERCHE EUROPEENNE**

0155871

Numéro de la demande

EP 85 40 0322

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | BULLETIN DES SOCIETES CHIMIQUES BELGES, vol. 85, no. 9, juillet 1976, pages 657-662,; L. VAN ROMPAEY et al.: "N-acylamino acids and peptides. V. Improved synthesis of N-ferulylglycyl-L-phenylalanine" * Pages 658-659, composé V * | 1,3,4 | C 07 C 69/734<br>C 07 D 213/42<br>C 07 D 277/04<br>C 07 C 67/327<br>C 07 C 67/00 |
| Y | EP-A-0 057 881 (KANEGAFUCHI KAGAKU KOGYO K.K.) * Page 25, revendication 1 * | 1,3 | |
| Y | FR-M- 4 305 (ELI LILLY AND COMPANY) * Pages 5,6; revendications * | 1,3 | |
| | | | **DOMAINES TECHNIQUES RECHERCHES (Int. Cl.4)** |
| | | | C 07 C 69/00<br>C 07 C 67/00 |

Le présent rapport de recherche a été établi pour toutes les revendications

| Lieu de la recherche | Date d'achèvement de la recherche | Examinateur |
|---|---|---|
| LA HAYE | 03-05-1985 | KINZINGER J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant